# Europäisches Patentamt

## European Patent Office

## Office européen des brevets

(19)

(11) Veröffentlichungsnummer: **0 090 223**
**B1**

(12) # EUROPÄISCHE PATENTSCHRIFT

(45) Veröffentlichungstag der Patentschrift:
**02.09.87**

(51) Int. Cl.⁴: **C 12 Q 1/00**

(21) Anmeldenummer: **83102388.2**

(22) Anmeldetag: **11.03.83**

(54) Testsystem und Verfahren zur Bestimmung von Substanzen in Flüssigkeiten.

(30) Priorität: **26.03.82 DE 3211167**

(43) Veröffentlichungstag der Anmeldung:
**05.10.83 Patentblatt 83/40**

(45) Bekanntmachung des Hinweises auf die Patenterteilung:
**02.09.87 Patentblatt 87/36**

(84) Benannte Vertragsstaaten:
**BE CH DE FR GB IT LI NL SE**

(56) Entgegenhaltungen:
**EP-A-0 089 606
DE-A-2 905 708
US-A-3 925 162
US-A-4 309 502**

(73) Patentinhaber: **Merck Patent Gesellschaft mit beschränkter Haftung, Frankfurter Strasse 250, D-6100 Darmstadt (DE)**

(72) Erfinder: **Limbach, Berthold, Dr., Otto- Hahn- Strasse 8, D-6104 Seeheim- Jugenheim (DE)**
Erfinder: **Helger, Roland, Dr., Ludwigshöhstrasse 85, D-6100 Darmstadt (DE)**

EP 0 090 223 B1

**Beschreibung**

Die Erfindung betrifft ein Testsystem und ein Verfahren mit erweitertem Meßbereich zur Bestimmung von Substanzen in Flüssigkeiten, insbesondere in Körperflüssigkeiten.

Die Bestimmung von Substanzen in Körperflüssigkeiten ist für die medizinische Diagnose von großer Bedeutung. Dies gilt sowohl für quantitative Bestimmungen als auch für semiquantitative oder qualitative Verfahren, die eine Selbstüberwachung des einzelnen Patienten oder ein einfaches Screening erlauben. Entsprechend der Bedeutung dieser Tests sind eine Vielzahl von Verfahren bekannt geworden. Die gebräuchlichsten Verfahren sind enzymatische Bestimmungsmethoden, die ein hohes Maß an Spezifität erlauben. Hier wird die Zu- oder Abnahme eines bei der Bestimmungsreaktion beteiligten Reaktanten mittelbar oder unmittelbar visuell, photometrisch oder mit anderen optischen oder physikalisch-chemischen Verfahren bestimmt. Allen diesen Systemen ist gemeinsam, daß immer nur ein Enzym oder Enzymsystem mit der zu bestimmenden Substanz reagiert, wobei im gleichen oder proportionalen Molverhältnis ein Umsetzungsprodukt gebildet oder ein Mitreaktant verbraucht wird. Infolgedessen ergibt sich für alle Verfahren ein in etwa gleich großer Meßbereich, der je nach Empfindlichkeit des Meßsignals und der Probenverdünnung verschoben sein kann.

Für die medizinische Diagnose sind Tests erforderlich, die in der Lage sind, sowohl nur wenig differierende Werte im Grenzbereich Normalbereich/pathologischer Bereich sicher zu erfassen, als auch gleichzeitig im pathologischen Bereich einen möglichst großen Bereich; abzudecken. Dies gilt insbesondere auch für Screening-Methoden, z. B. mit Teststäbchensystemen. Der für die üblichen Tests erhaltene Meßbereich ist für einen Teil der anfallenden Aufgaben nicht ausreichend; dies trifft vor allem für die Bestimmung von Glucose zu. Es ist zwar schon versucht worden, dieses Problem durch Testzusätze zu lösen, die die Testempfindlichkeit verändern; ein größerer Meßbereich wird aber nur dann erhalten, wenn mehrere Tests mit entsprechend verschieden eingestellten Meßbereichen in den Gesamttest einbezogen werden.

Der Erfindung liegt die Aufgabe zugrunde, Testsysteme und Verfahren zu entwickeln, die gegenüber den üblichen Systemen und Verfahren einen vielfach größeren Meßbereich bei mindestens gleicher Meßgenauigkeit haben. Gelöst wurde diese Aufgabe dadurch, daß man mehrere verschiedene Enzyme oder Enzymsysteme, die das gleiche Substrat als Ausgangsreaktant unabhängig voneinander umsetzen, aber verschiedene unterscheidbare Endprodukte ergeben, miteinander koppelt.

Gegenstand der Erfindung ist ein Testsystem zur Bestimmung von Substanzen in Flüssigkeiten, daß dadurch gekennzeichnet ist, daß es mehrere Enzymes oder Enzymsysteme enthält, die mit jeweils einer einzigen zu bestimmenden Substanz unter Bildung analytisch unterscheidbarer Endprodukte reagieren.

Ein weitere Gegenstand der Erfindung sind Verfahren zur Bestimmung von Substanzen in Flüssigkeiten, die dadurch gekennzeichnet sind, daß die Probelösung mit mehreren Enzymen oder Enzymsystemen behandelt wird, wobei verschiedene analytisch unterscheidbare Endprodukte entstehen, die meßtechnisch oder visuell ausgewertet werden.

Eine sequenzielle Koppelung von verschiedenen enzymatischen Reaktionen ist in einer Vielzahl von Beispielen bekannt, wie die Hexokinase/Glucose-6-phosphatdehydrogenase-Reaktion zur Glucosebestimmung oder die Urease/Glutamatdehydrogenase-Reaktion zur Harnstoffbestimmung. Diesen Enzymsystemen ist gemeinsam, daß sie hintereinandergeschaltet sind, d.h. das Reaktionsprodukt der ersten Reaktion ist Ausgangssubstrat der zweiten Reaktion. Es ist auch bekannt, mehrere Enzyme durch Zugabe der entsprechenden Substrate in einer einzigen Probelösung zu bestimmen; die Auswertung erfolgt jedoch wie üblich bei der jeweils für die einzelne Bestimmung charakteristischen Wellenlänge (US-A-3 925 162).

Eine parallele Koppelung von verschiedenen enzymatischen Reaktionen, d.h. das gleichzeitige Vorliegen mehierer, das gleiche Substrat umsetzender Enzymsysteme müßte zu einer Konkurrenz der beiden Reaktionen führen und damit zu einer gegenseitigen Abhängigkeit der Reaktionen. So erhält man etwa bei der Koppelung der Glucoseoxidase- mit der $NAD^+$-abhängigen Glucosedehydrogenase-Reaktion einen gleichzeitigen Ablauf beider Reaktionen; das Verhältnis der dabei jeweils gebildeten Endprodukte ist von der jeweiligen Konzentration der beiden Enzyme abhängig. Erhöht man z. B. die Konzentration an Glucoseoxidase, so wird unter sonst gleichen Bedingungen bei gleicher Glucosekonzentration weniger $NAD^+$ reduziert. Insgesamt ergibt sich auf diese Weise zwar eine Vergrößerung des Meßbereiches, dies aber auf Kosten der Meßgenauigkeit. Darüberhinaus beschreiben beide Reaktionen einen gemeinsamen Meßbereich.

Umso überraschender ist es, daß eine Koppelung mehrerer, das gleiche Substrat unabhängig voneinander direkt oder indirekt zu verschiedenen Produkten umsetzender Enzymsysteme möglich Ist, wobei mindestens zwei verschiedene Meßsignale in einem Reaktionsansatz erhalten werden, die jeweils zwei verschiedene Konzentrationsbereiche der zu bestimmenden Substanz beschreiben. Unabhängig voneinander bedeutet dabei, daß bei gleichzeitigem Vorliegen der die Substanz umsetzenden erfindungsgemäßen Systeme die Umsetzung durch das zweite System erst nach weitgehendem Verbrauch des Coenzyms des ersten Systems erfolgt. Man erhält dadurch zwei definierte Meßbereiche in einem Gesamtmeßbereich.

Die erfindungsgemäßen Enzyme oder Enzymsysteme sind Dehydrogenasen mit voneinander unabhängigen Elektronenakzeptoren bzw. -donatoren. Das Testsystem enthält demnach folgende Komponenten:

a) Ein Enzym oder Enzymsystem, das reduziertes oder oxidiertes Nicotinamidadenin-dinucleotid (NADH, $NAD^+$) bzw. Nicotinsäureamidadenin-dinucleotidphosphat (NADPH, $NADP^+$) umsetzt.

b) Ein Enzym oder Enzymsystem, das die unter a) genannten Coenzyme nicht umsetzt, sondern einen Elektronenakzeptor bzw. -donator, der ein von diesen Coenzymen unterscheidbares Meßsignal liefert. Solche Substanzen sind z. B. bekannt als Coenzyme aus der Klasse der Cytochrome, Quinone oder Fyrroloquinolinquinone; in Frage kommen auch Flavinnucleotide, Hexacyanoferrat, Methylenblau, Phenazinmethosulfat, Phenazinethosulfat, Benzochinon, Dichlorphenolindophenol, Dichlorindophenol, Trichlorindophenol und andere. Entsprechende, diese Coenzyme umsetzende Enzyme sind aus der Literatur bekannt.

c) Alle für die Durchführung eines diagnostischen Tests mit Hilfe des erfindungsgemäßen Enzymsystems notwendigen Coenzyme und Hilfsubstanzen wie Puffer, Stabilisierungsmittel, Chromogene usw.

Durch Koppelung von jeweils einem der unter a) und b) genannten Enzyme bzw. Enzymsysteme erhält man ein erfindungsgemäßes Testsystem mit zwei definierten Meßbereichen, die sich zu einem erweiterten Gesamtmeßbereich ergänzen. In der nachstehenden Tabelle ist beispielhaft eine Auswahl von mit Hilfe des Testsystems bestimmbarer Substanzen aufgeführt, die beliebig erweitert werden kann. Die für die NAD-abhängigen und nicht-NAD-abhängigen Enzyme angegebenen Nummern entsprechen der Enzymnomenklatur des Nomenklatur Committee of the International Union of Biochemistry:

| zu bestimmende Substanz | NAD-abhängiges Enzym | nicht-NAD-abhängigens Enzym |
| --- | --- | --- |
| Alkohol | 1.1.1.1 | 1.1.99.8 |
| Glucose | 1.1.1.47 | 1 1 99. - |
| Glycerin-3-phosphat | 1.1.1.8 | 1.1.99.5 |
| Glycin | 1.4.1.10 | 1.4.2.1 |
| Laktat | 1.1.1.27 | 1.1.2.3 |
| Malat | 1.1.1.37 | 1.1.99.16 |
| Mannitol | 1.1.1.67 | 1.1.2.2 |

Im Falle der Bestimmung von Glucose ist das NAD-abhängige Enzym Glucosedehydrogenase 1.1.1.47, vorzugsweise aus Bacillus megaterium, und das nicht-NAD-abhängige Enzym Glucosedehydrogenase 1.1.99., vorzugsweise aus Acinetobacter calcoaceticus. Für Glucosebestimmungen mit Systemen, die jeweils nur eine (NAD-abhängige bzw. nicht-NAD-abhängige) Glucosedehydrogenase enthalten, ergibt sich bei gleicher Probenverdünnung eine verschiedene Lage, aber in etwa gleicher Umfang des Meßbereichs. Das aus beiden Enzymsystemen gekoppelte System ermöglicht dagegen einen bedeutend größeren Meßbereich. Eine Koppelung von Glucosedehydrogenase und Glucoseoxidase führt zwar ebenfalls zu einer Erweiterung des Meßbereiches; dabei ergibt sich aber aus der gegenseitigen Konkurrenz die erwartete Verringerung der Meßgenauigkeit.

Das NAD-umsetzende Enzymsystem kann zusätzlich ein Tetrazoliumsalz und ein darauf elektronenübertragendes System, z. B. Diaphorase, enthalten.

Die beschriebenen Enzyme setzen in der Regel die zu bestimmende Substanz direkt um. Es ist aber auch möglich, das erfindungsgemäße, gekoppelte Enzymsystem mit einer oder mehreren vorangegangenen Enzymreaktionen zu koppeln. Als Beispiel sei folgendes Reaktionssystem aufgeführt:

$$\text{Triglyceride} \xrightarrow{\text{Lipase}} \text{Glycerin}$$

$$\text{Glycerin} \xrightarrow{\text{Glycerokinase}} \text{Glycerin-3-phosphat}$$

$$\text{Glycerin-3-phosphat} + \text{NAD}^+ \xrightarrow{\text{Glycerin-3-phosphatdehydrogenase}} \text{NADH} + \text{Dihydroxyacetonphosphat}$$

$$\text{Akzeptor} \xrightarrow{\text{Glycerin-3-phosphatdehydrogenase}} \text{red. Akzeptor} + \text{Dihydroxyacetonphosphat}$$

Damit ist es möglich, Testverfahren mit erweitertem Meßbereich für eine Vielzahl von Bestimmungen durchzuführen.

Ebenso ist es mit Hilfe des erfindungsgemäßen Testsystems möglich, ein Substrat umzusetzen, das durch Umsetzung eines zu bestimmenden Enzyms in einer Reaktion gebildet wird. Hierbei lassen sich insbesondere auf einfache Weise Störeinflüsse, wie additive Blindwerte, eliminieren. So kann etwa bei der Amylase bestimmung die endogene Glucose in einer ersten Reaktion beseitigt werden, während der durch die zweite Reaktion bedingte Meßbereich voll zur Bestimmung der gebildeten Glucose erhalten bleibt.

Das erfindungsgemäße Testsystem bietet auch die Möglichkeit, einen Meßbereich zu verändern, während der zweite unverändert bleibt. Damit kann man das erfindungsgemäße Testsystem derart beeinflussen, daß für den beschriebenen Grenzbereich zwischen Normalbereich und pathologischem Bereich ein den üblichen Methoden entsprechender Meßbereich existiert, während für den pathologischen Bereich ein variabler, an die Anforderungen der medizinischen Diagnose anpaßbarer Meßbereich gestaltet werden kann. Im Falle einer Glucosebestimmung wird dies durch Zusatz von Glucoseoxidase und Peroxidase erreicht.

Die Durchführüng der enzymatischen Bestimmung mit Hilfe des erfindungsgemäßen Testsystems erfolgt in der für enzymatische Methoden übliche Weise, wobei jedoch beide Enzymsysteme gleichzeitig zur Probelösung gegeben werden. Die Auswertung kann visuell, photometrisch oder mit anderen optischen wie auch physikalisch-chemischen Methoden erfolgen. Bei photometrischer Bestimmung kann die Auswertung nach dem Endpunktverfahren bei zwei definierten Wellenlängen erfolgen (z. B. bei 334 und 600 nm oder bei 460 und 650 nm). Ebenso ist es möglich, kinetisch bei einer oder zwei Wellenlängen den Reaktionsverlauf zu registrieren und auszuwerten. Der erweiterte Meßbereich ermöglicht dabei vornehmlich eine einfachere Bestimmung auch erhöhter pathologischer Werte für eine Substanz; eine zusätzliche Verdünnung ist nicht nötig.

Die Erfindung wird durch die nachfolgenden Beispiele näher erläutert:

**Beispiel 1**

Bestimmung von Glucose mit einem gekoppelten System aus Glucosedehydrogenase mit NAD als Coenzym und Glucosedehydrogenase mit Dichlorphenolindophenol als Coenzym.

Es werden je 1 ml der folgenden Reaktionsgemische, enthaltend

a) 0,12 mol/l Phosphatpuffer, pH 6,5,
1,0 mmol/l NAD+
, 5,0 kU/l Glucosedehydrogenase aus Bacillus megaterium

b) 0,12 mol/l Phosphatpuffer, pH 6,5,
0,12 mmol/l Dichlorphenolindophenol,
250 U/l Glucosedehydrogenase aus Acinetobacter calcoacetius

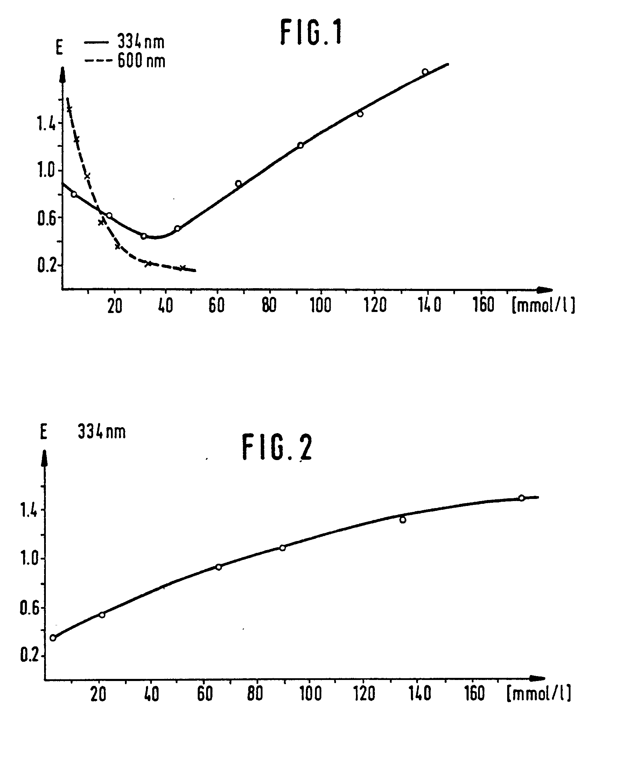

miteinander gemischt und mit 40 µl Serum (1 + 1 enteiweißt mit 0,3 mol/l Trichloressigsäure) mit unterschiedlichem definiertem Glucosegehalt versetzt. Nach jeweils 15 Minuten wird die Extinktion bei 334 und 600 nm gemessen. Die erhaltenen Ergebnisse sind in Abbildung 1 dargestellt.

Während mit den beiden Einzelreaktionen im einem Fall Glucosebestimmungen von 0,5 - 12 mmol/l und im anderen Fall von etwa 2 - 60 mmol/l durchgeführt werden können, zeigt die Abbildung 1, daß mit dem erfindungsgemäßen gekoppelten System Glucose von 0,5 bis über 160 mmol/l sicher bestimmt werden kann. 1 mmol/l entspricht 18 mg-% Glucose.

**Beispiel 2**

Bestimmung von Glucose mit Glucosedehydrogenase/NAD in Gegenwart von Glucoseoxidase und Peroxidase.

Es werden die Bedingungen von Beispiel 1 und der Ansatz nach 1a) verwendet. Das Reaktionsgemisch enthält zusätzlich 1 kU/l Glucoseoxidase und 400 U/l Peroxidase. Die erhaltene Meßkurve ist in Abbildung 2 dargestellt.

Die Abbildung zeigt, daß die Koppelung von Glucosedehydrogenase- und Glucoseoxidasereaktion ebenfalls zu einer Erweiterung des Meßbereichs führt, wobei jedoch aufgrund des flachen Kurvenverlaufs die Meßgenauigkeit verringert ist.

**Beispiel 3**

Visuelle Bestimmung von Glucose mit einem gekoppelten System aus Glucosedehydrogenase/NAD/Diaphorase/Tetrazoliumsalz und Glucosedehydrogenase/Dichlorphenolindophenol; Variation des Meßbereichs

Es werden je 2 ml eines Reaktionsgemisches vier verschiedener Ansätze verwendet, die alle 0,12 mol/l Phosphatpuffer, pH 6,5, und 10⁻³ Gew.-% Fluoreszein-Natrium enthalten. Darüberhinaus enthält

Ansatz A:  1 mmol/l NAD⁺,
0,67 mmol/l 3-(4-Jodphenyl)-2-(4-nitrophenyl)-5-phenyl-2H- -tetrazoliumchlorid (INT),
5 kU/l Glucosedehydrogenase aus B. mega- terium und
250 U/l Diaphorase.

Ansatz B 1:  1 mmol/l NAD⁺,
0,25 mmol/l Dichlorphenolindophenol,
0,67 mmol/l INT,
2,5 kU/l Glucosedehydrogenase aus B. megaterium,
250 U/l Glucosedehydrogenase aus A. calcoaceticus und
250 U/l Diaphorase.

Ansatz B 2:  die im Ansatz B 1 aufgeführten Reagenzien in der gleichen Konzentration
zuzüglich 500 U/l Glucoseoxidase und
200 U/l Peroxidase

Ansatz B 3:  die im Ansatz B 1 aufgeführten Reagenzien in den gleichen Konzentrationen
zuzüglich 1 kU/l Glucoseoxidase und
400 U/l Peroxidase.

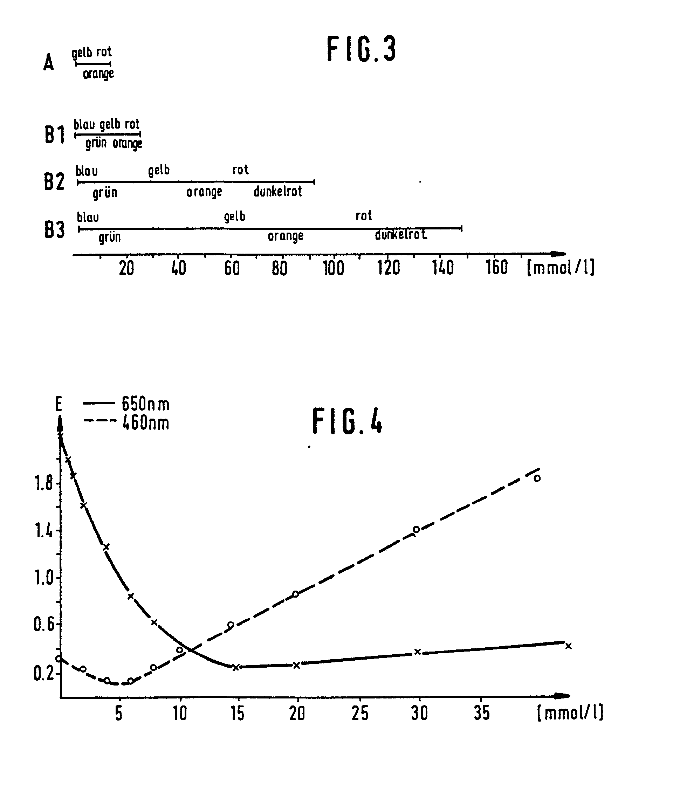

Zu dem Reaktionsgemisch werden jeweils 60 µl Serum (1 + 1 enteiweißt mit 0,3 mol/l Trichloressigsäure) mit unterschiedlichem definiertem Glucosegehalt pipettiert. Nach 20 Minuten wird visuell der Farbeindruck der Gesamtlösung beurteilt; die visuell unterscheidbaren Glucosekonzentrationen ergeben dabei den Gesamtmeßbereich. In Abbildung 3 ist das Ergebnis graphisch dargestellt. Man erkennt deutlich den größeren Meßbereich des gekoppelten System sowie die mögliche breite Varianz.

**Beispiel 4:**

Bestimmung von Laktat mit einem gekoppelten System aus Laktatdehydrogenase/NAD/Diaphorase und Laktatdehydrogenase/Dichlorphenolindophenol.
Es werden 2 ml eines Reaktionsgemisches verwendet, das
0,25 mol/l Phosphatpuffer, pH 7,0,
1 mmol/l NAD⁺,
0,12 mmol/l Dichlorphenolindophenol,
0,67 mmol/l INT,
5 kU/l Laktatdehydrogenase aus Schweineherz,
250 U/l Diaphorase und
200 U/l Laktatdehydrogenase aus Hefe
enthält. Dazu werden 100 µl Serum (1 + 1 enteiweißt mit 0,3 mol/l Trichloressigsäure) pipettiert. Nach jeweils 30 Minuten wird die Extinktion bei 460 und 650 nm gemessen. Die erhaltenen Ergebnisse sind in Abbildung 4 dargestellt.
Während mit den beiden Einzelreaktionen in einem Fall Laktatbestimmungen von 0,2 - 6 mmol/l und im anderen Fall von etwa 0,8 - 24 mmol/l durchgeführt werden können, zeigt die Abbildung, daß mit dem erfindungsgemäßen gekoppelten System Laktat von 0,2 bis über 60 mmol/l bestimmt werden kann.

**Beispiel 5**

Bestimmung von Alhohol mit einem gekoppeiten System aus Alkoholdehydrogenase/NAD/Diaphorase und Alkoholdehydrogenase/Dichlorphenolindophenol.
Es werden 2 ml eines Reaktionsgemisches verwendet, das
0,3 mol/l Tris(hydroxymethyl)-aminomethan-Puffer, pH 8,0,
1 mmol/l NAD⁺,
0,12 mmol/l Dichlorphenolindophenol,
0,67 mmol/l INT,
2 kU/l ADH aus Hefe,
250 U/l Diaphorase und
250 U/l ADH aus Pseudomonas sp. M 27
enthält. Dazu werden 50 µl Serum (1 + 1 enteiweißt mit 0,3 mol/l Trichloressigsäure) pipettiert. Nach jeweils

5

30 Minuten wird die Extinktion bei 460 und 650 nm gemessen. Es ergibt sich ein Meßbereich von 0,02 bis über 3 g/l Alkohol, während mit den beiden Einzelreaktionen lediglich Meßbereiche von 0,02 bis 0,5 bzw. von 0,08 bis 2,2 g/l Alkohol erhältlich sind.

**Beispiel 6**

Bestimmung von Glucose im Urin mit Hilfe von Teststreifen

Ein saugfähiges Material wird mit Lösungen analog Beispiel 3, jedoch 20-fach höherer Enzymkonzentration getränkt, vorsichtig getrocknet und auf einem geeigneten Trägermaterial fixiert. Nach Eintauchen in die Probelösung erhält man je nach dem Glucosegehalt Farbumschläge von Blau über Blaugrün, Grün, Hellgrün, Beige, Orange, Rot bis Dunkelrot. Die Auswertung kann durch Vergleich mit einer geeigneten Standardfarbskala erfolgen.

**Patentansprüche**

1 Testsystem zur Bestimmung von Substanzen in Flüssigkeiten, dadurch gekennzeichnet, daß es mehrere Enzyme oder Enzymsysteme enthält, die mit jeweils einer einzigen zu bestimmenden Substanz unter Bildung analytisch unterscheidbarer Endprodukte reagieren.

2. Testsystem nach Anspruch 1, dadurch gekennzeichnet, daß die Enzyme oder Enzymsysteme Dehydrogenasen mit voneinander unabhängigen Elektronenakzeptoren bzw. -donatoren sind.

3. Testsystem nach den Ansprüchen 1 und 2, dadurch gekennzeichnet, daß eines der Enzymsysteme eine reduzierte oder oxidierte Nicotinamidadenindinucleotid bzw. -dinucleotidphosphat umsetzende Dehydrogenase und das andere eine diese Coenzyme nicht umsetzende Dehydrogenase mit einem anderen Elektronenakzeptor bzw. -donator entält.

4. Testsystem zur Bestimmung von Glucose nach den Ansprüchen 1 bis 3, dadurch gekennzeichnet, daß das NAD umsetzende Enzymsystem Glucosedehydrogenase aus Bacillus megaterium und das nicht NAD umsetzende Enzymsystem Glucosedehydrogenase aus Acinetobacter calcoaceticus enthält.

5. Testsystem nach Ansprüchen 1 bis 4, dadurch gekennzeichnet, daß das NAD umsetzende Enzymsystem zusätzlich ein Tetrazoliumsalz und ein darauf elektronenübertragendes System enthält.

6. Testsytem nach den Ansprüchen 4 und 5, dadurch gekennzeichnet, daß es zusätzlich Glucoseoxidase und Peroxidase enthält.

7. Verfahren zur Bestimmung von Substanzen in Flüssigkeiten, dadurch gekennzeichnet, daß die Probelösung mit mehreren Enzymen oder Enzymsystemen behandelt wird, wobei verschiedene analytisch unterscheidbare Endprodukte entstehen, die meßtechnisch oder visuell ausgewertet werden.

**Claims**

1. Test system for the determination of substances in liquids, characterised in that it contains several enzymes or enzyme systems which independently of one another, react directly or indirectly with the substance to be determined.

2. Test system according to Claim 1, characterised in that the enzymes or enzyme systems are dehydrogenases with electron acceptors or donors which are independent of one another.

3. Test system according to Claims 1 and 2, characterised in that one of the enzyme systems contains a dehydrogenase which converts a reduced or ooxidised nicotinamide-adenine dinucleotide or dinucleotide phosphate, and the other contains a dehydrogenase, which does not convert these coenzymes, with another electron acceptor or donor.

4. Test system for the determination of glucose according to Claims 1 to 3, characterised in that the enzyme system converting NAD contains glucose dehydrogenase from Bacillus megaterium and the enzyme system not converting NAD contains glucose dehydrogenase from Acinetobacter calcoaceticus.

5. Test system according to Claims 1 to 4, characterised in that the enzyme system converting NAD also contains a tetrazolium salt and a system transferring electrons thereto.

6. Test system according to Claims 4 and 5, characterised in that it also contains glucose oxidase and peroxidase.

7. Procedure for the determination of substances in liquids, characterised in that the sample solution is treated simultaneously with several enzymes or enzyme systems which, independently of one another, react directly or indirectly with the substance to be determined, there being produced various final products which can be distinguished analytically and evaluated by measurement techniques or visually.

## Revendications

1. Système de testage pour la détermination de substances dans des liquides, caractérisé en ce qu'il contient plusieurs enzymes ou systèmes d'enzymes qui réagissent avec chaque fois avec une seule substance à déterminer tout en formant des produits finals différenciables analytiquement.

2. Système de testage selon la revendication 1, caractérisé en ce que les enzymes ou systèmes d'enzymes sont des déshydrogénases avec des accepteurs ou donateurs d'électrons indépendants les une des autres.

3. Système de testage selon les revendications 1 et 2, caractérisé en ce qu'un des systèmes d'enzymes contient une déshydrogénase entrant en réaction avec un dinucléotide ou dinucléotide-phosphate de nicotinamide-adénine réduit ou oxydé et l'autre une déshydrogénase n'entrant pas en réaction avec ces coenzymes, de même qu'un autre accepteur ou donateur d'électrons.

4. Système de testage pour la détermination du glucose selon les revendications 1 à 3, caractérisé en ce que le système d'enzymes réagissant avec le NAD contient de la glucose déshydrogénase de Bacillus megaterium et en ce que le système d'enzymes ne réagissant pas avec le NAD contient de la glucose déshydrogénase d'Acinetobacter calcoaceticus.

5. Système de testage selon les revendications 1 à 4, caractérisé en ce que le système d'enzymes réagissant avec le NAD contient en plus un sel de tétrazolium et un système lui transmettant des électrons.

6. Système de testage selon les revendications 4 et 5, caractérisé en ce qu'il contient en plus de la glucose oxydase et de la peroxydase.

7. Procédé de détermination de substances dans des liquides, caractérisé en ce qu'on traite la solution de la prise d'essai avec plusieurs enzymes ou systèmes d'enzymes, avec obtention de différents produits finals différenciables analytiquement pouvant être évalués par une technique de mesure ou visuellement.

FIG.1

FIG.2

# FIG.3

A — gelb rot / orange

B1 — blau gelb rot / grün orange

B2 — blau gelb rot / grün orange dunkelrot

B3 — blau gelb rot / grün orange dunkelrot

20  40  60  80  100  120  140  160  [mmol/l]

# FIG.4

E — 650nm (solid line) / 460nm (dashed line)

1.8 | 1.4 | 1.0 | 0.6 | 0.2

5  10  15  20  25  30  35  [mmol/l]